# EUROPEAN PATENT APPLICATION

(11) **EP 3 513 722 A1**
(43) Date of publication of application: **24.07.2019**
(21) Application number: 19152142.6
(22) Date of filing: 16.01.2019
(51) Int. Cl.: A61B 5/055

(54) **MAGNETIC RESONANCE IMAGING APPARATUS**

(30) Priority: 17.01.2018 KR 20180006119
(71) Applicant: Samsung Electronics Co., Ltd., Suwon-si, Gyeonggi-do, 16677 (KR)
(72) Inventor: JEONG, ManHo, 16677 Gyeonggi-do (KR); CHAE, YoungJae, 16677 Gyeonggi-do (KR)
(74) Representative: Grootscholten, Johannes A.M.

(57) **Abstract**

A magnetic resonance imaging apparatus includes a magnet assembly; a base disposed adjacent to the magnet assembly; a seating member on which an object is to be seated and configured to be rotatably disposed on the base; and an elastic assembly configured to elastically support the seating member on the base to compensate for a load of the object. The elastic assembly includes: an elastic unit having a first end connected to the seating member and configured to generate an elastic force; and a control device disposed on the base and configured to guide a second end opposite to the first end of the elastic unit to adjust an angle between the seating member and the elastic unit.

## Description

### BACKGROUND

### 1. Technical Field

The present disclosure relates to a magnetic resonance imaging apparatus, and more particularly, to a magnetic resonance imaging apparatus with improved convenience.

### 2. Description of the Related Art

In the related art, a medical imaging apparatus is a type of apparatus used for acquiring graphical information of a patient and providing an relevant image of the patient. Medical imaging apparatuses include an X-ray apparatus, an ultrasonic diagnostic apparatus, a computerized tomography apparatus, a magnetic resonance imaging apparatus and the like.

Amongst these apparatuses, the magnetic resonance imaging apparatus has an important role in the field of diagnosis or treatment monitoring using medical images because the magnetic resonance imaging apparatus is relatively independent of imaging conditions and provides excellent contrast and diagnostic information on various images of soft tissues of the patient.

Magnetic Resonance Imaging (MRI) is the imaging of the atomic nucleus density and physico-chemical properties by generating a nuclear magnetic resonance phenomenon on a hydrogen nucleus in a human body using a magnetic field free from harm to the human body and radio frequency (RF) which is non-ionizing radiation.

Specifically, the magnetic resonance imaging apparatus supplies a constant frequency and energy to a cavity in a state of applying a constant magnetic field, converts energy emitted from an atomic nucleus into a signal, and generates images the interior of an object.

The magnetic resonance imaging apparatus can acquire clinically useful images when a specific part of a patient is fixed without moving for a certain period of time. In this case, the cradle or chair on which the patient is positioned should be configured to maintain the patient in a comfortable posture so that the patient does not move for a certain period of time as required to generate clear MRI images. However, because the patient's physical condition, size and weight may vary, there is a need for an apparatus that can be freely adjusted to accommodate various patients with different physical condition, size and weight.

### SUMMARY

It is an aspect of the present disclosure to provide a magnetic resonance imaging apparatus having a seating structure which facilitates the posture correction of an object (a patient).

It is another aspect of the present disclosure to provide a magnetic resonance imaging apparatus having a structure capable of compensating for a load according to an object.

Additional aspects of the present disclosure will be set forth in part in the description which follows and, in part, will be obvious from the description, or may be learned by practice of the disclosure.

In accordance with an aspect of the present disclosure, a magnetic resonance imaging apparatus includes a magnet assembly having a cavity, a base disposed adjacent to the magnet assembly, a seating member on which an object is seated and configured to be rotatably disposed on the base to insert the object into the cavity, and an elastic assembly configured to elastically support the seating member on the base to compensate for a load in the direction of gravity of the object, wherein the elastic assembly includes an elastic unit having one end connected to the seating member and generating an elastic force, and a control device disposed on the base and configured to be capable of moving the other end of the elastic unit to adjust an angle between the seating member and the elastic unit.

The control device may be configured to be capable of adjusting the angle according to a weight of the object.

The elastic unit may be configured to be rotatable about a first rotation shaft with respect to the seating member and to be rotatable about a second rotation shaft with respect to the control device, and the control device may be configured to guide the elastic unit so that the second rotation shaft moves in a circumferential direction about the first rotation shaft.

The elastic unit may include a moving shaft forming the second rotation shaft, and the control device may include a device body disposed on the base, and a shaft guide formed in the device body to guide circumferential movement of the moving shaft.

The elastic unit may further include a control handle connected to the moving shaft, and the control device may further include a plurality of handle fixing portions formed to extend from the shaft guide and to which the control handle is fixed to restrict movement of the moving shaft.

The plurality of handle fixing portions may be formed radially from the shaft guide and disposed to be spaced apart from each other.

The shaft guide may be formed in a shape of an arc in which the first rotation shaft is a center of the radius of curvature.

The first rotation shaft may move together with the rotation of the seating member, and the control device may be configured to rotate in conjunction with the movement of the first rotation shaft.

The control device may be interlocked with the first rotation shaft so that the shaft guide maintains the first rotation shaft, which moves, to be a center of the radius of curvature.

The control device may include a device rotation shaft forming a rotation center of the device body, and an interlocking protrusion spaced apart from the device rotation shaft and formed integrally with the device body, and the magnetic resonance imaging apparatus may further include an interlocking member having one end connected to the seating member so as to be rotatable about the same axis as the first rotation shaft and whose rotation is restricted by the device rotation shaft and the interlocking protrusion, wherein the interlocking member may transmit the movement of the seating member to the interlocking protrusion to rotate the control device.

The interlocking member may include a member body rotatably provided on the seating member about the same axis as the first rotation shaft and formed in a bar shape, and an interlocking guide forming a hole in a longitudinal direction of the member body and provided such that the device rotation shaft and the interlocking protrusion are positioned therein.

The shaft guide may include a stopper surface for restricting movement of the moving shaft in a radial direction with respect to the first rotation shaft.

The seating member may include a sitting plate on which the object sits, a backrest disposed to form a predetermined angle with the sitting plate, and a connecting member for connecting the sitting plate and the backrest, and a first rotation shaft may be formed in the connecting member.

The base may be configured to rotatably support a front portion of the seating member, and the elastic unit may be configured to elastically support a rear portion of the seating member.

The elastic unit may include a gas spring.

In accordance with another aspect of the present disclosure, a magnetic resonance imaging apparatus includes a base, a seating member on which an object is seated and configured to be rotatably disposed on the base, an elastic unit having one end elastically supporting the seating member, and a control device disposed on the base to support the other end of the elastic unit, wherein the control device includes a device body provided to rotate in conjunction with the rotation of the seating member on the base, and a shaft guide provided on the device body to guide the movement of the other end of the elastic unit in order to adjust an angle between the seating member and the elastic unit according to the weight of the object.

The elastic unit may be configured to be rotatable about a first rotation shaft with respect to the seating member and to be rotatable about a second rotation shaft with respect to the control device, and the shaft guide may be formed in a shape of an arc in which the first rotation shaft is a center of the radius of curvature.

The first rotation shaft may move together with the rotation of the seating member, and the control device may be interlocked with the first rotation shaft so that the shaft guide maintains the first rotation shaft, which moves, to be a center of the radius of curvature.

In accordance with another aspect of the present disclosure, a magnetic resonance imaging apparatus includes a magnet assembly having a cavity, a base disposed adjacent to the magnet assembly, a seating member on which an object is seated and configured to be rotatably disposed on the base to insert the object into the cavity, an elastic unit having one end connected to the seating member so as to be rotatable about a rotation shaft integrally formed with the seating member and configured to elastically support the seating member, a control device having a shaft guide formed in a shape of an arc in which the rotation shaft is a center of the radius of curvature and configured to move the other end of the elastic unit along the shaft guide to adjust an angle between the seating member and the elastic unit, and an interlocking member configured to be interlocked with the movement of the seating member to rotate the control device so that the shaft guide maintains a center of the radius of curvature.

The elastic unit may be configured to be rotatable about a first rotation shaft with respect to the seating member and to be rotatable about a second rotation shaft with respect to the control device, the control device may include a device body disposed on the base, a device rotation shaft forming a rotation center of the device body, and an interlocking protrusion spaced apart from the device rotation shaft and formed integrally with the device body, and the interlocking member may include a member body rotatably provided on the seating member about the same axis as the first rotation shaft and formed in a bar shape, and an interlocking guide forming a hole in a longitudinal direction of the member body and provided such that the device rotation shaft and the interlocking protrusion are positioned therein.

In accordance with another aspect of the present disclosure, a magnetic resonance imaging apparatus includes: a magnet assembly; a base disposed adjacent to the magnet assembly; a seating member on which an object is to be seated and configured to be rotatably disposed on the base; and an elastic assembly configured to elastically support the seating member on the base to compensate for a load of the object. The elastic assembly includes: an elastic unit having a first end connected to the seating member and configured to generate an elastic force; and a control device disposed on the base and configured to guide a second end opposite to the first end of the elastic unit to adjust an angle between the seating member and the elastic unit.

The control device may be configured to adjust the angle between the seating member and the elastic unit according to a weight of the object.

The elastic unit may be configured to be rotatable about a first rotation shaft with respect to the seating member and to be rotatable about a second rotation shaft with respect to the control device, and the control device may be configured to guide the elastic unit so that the second rotation shaft moves in a circumferential direction about the first rotation shaft.

The second rotation shaft of the elastic unit may include a moving shaft. The control device may include: a device body disposed on the base; and a shaft guide formed in the device body to guide the moving shaft.

The elastic unit may further include a control handle connected to the moving shaft, and the control device may further include a plurality of handle fixing portions extending from the shaft guide and into which the control handle is fixed to restrict movement of the moving shaft.

The plurality of handle fixing portions may be formed as slots in the device body and disposed to be spaced apart from one another.

The shaft guide may have an arc shape in which the second rotation shaft is located at a center of radius of curvature of the arc shape.

The first rotation shaft may move together with the seating member, and the control device may be configured to rotate in conjunction with a movement of the first rotation shaft.

The control device may be interlocked with the second rotation shaft so that the shaft guide maintains the second rotation shaft, which moves, to be a center of radius of curvature of the shaft guide.

The control device may include: a device rotation shaft attached to the base and forming a rotation center of the device body; and an interlocking protrusion spaced apart from the device rotation shaft and formed integrally with the device body. The magnetic resonance imaging apparatus may further include an interlocking member having one end connected to the seating member so as to be rotatable about an axis of the first rotation shaft, a rotation of the interlocking member being restricted by the device rotation shaft and the interlocking protrusion, and the interlocking member may be configured to transmit a force generated by a movement of the seating member to the interlocking protrusion to rotate the control device.

The interlocking member may include: a member body rotatably provided on the seating member about the axis of the first rotation shaft and formed in a bar shape; and an interlocking guide including a hole extending in a longitudinal direction of the member body, the device rotation shaft and the interlocking protrusion being disposed in the interlocking guide.

The shaft guide may include a stopper surface for restricting a movement of the moving shaft in an axial direction of the first rotation shaft.

The seating member may include: a sitting plate on which the object sits; a backrest disposed to form a predetermined angle with the sitting plate; and a connecting member connecting the sitting plate and the backrest, and the first rotation shaft is provided in the connecting member.

The base may be configured to rotatably support a first portion of the seating member, and the elastic unit may be configured to elastically support a second portion opposite to the first portion of the seating member.

The elastic unit may include a gas spring.

In accordance with another aspect of the present disclosure, a magnetic resonance imaging apparatus may include: a base; a seating member on which an object is to be seated and configured to be rotatably disposed on the base; an elastic unit having a first end elastically supporting the seating member; and a control device disposed on the base to support a second end opposite to the first end of the elastic unit. The control device may include: a device body provided to rotate in conjunction with rotation of the seating member with respect to the base; and a shaft guide provided on the device body to guide movement of the second end of the elastic unit in order to adjust an angle between the seating member and the elastic unit according to a weight of the object.

The elastic unit may be configured to be rotatable about a first rotation shaft with respect to the seating member and to be rotatable about a second rotation shaft with respect to the control device, and the shaft guide may have a shape of an arc in which the second rotation shaft is located at a center of a radius of curvature of the arc.

The first rotation shaft may move together with the rotation of the seating member, and the control device may be interlocked with the second rotation shaft so that the shaft guide maintains the second rotation shaft, which moves, to be at the center of the radius of curvature in the shaft guide.

In accordance with another aspect of the present disclosure, a magnetic resonance imaging apparatus may include: a magnet assembly; a base disposed adjacent to the magnet assembly; a seating member on which an object is to be seated and configured to be rotatably disposed on the base; an elastic unit having a first end connected to the seating member so as to be rotatable about a first rotation shaft integrally formed with the seating member and configured to elastically support the seating member; a control device having a shaft guide formed in a shape of an arc in which a second rotation shaft is at a center of a radius of curvature of the shaft guide and configured to move a second end opposite to the first end of the elastic unit along the shaft guide to adjust an angle between the seating member and the elastic unit; and an interlocking member configured to be interlocked with a movement of the seating member to rotate the control device so that the shaft guide maintains the second rotation shaft at the center of the radius of curvature.

The elastic unit may be configured to be rotatable about the first rotation shaft with respect to the seating member and to be rotatable about the second rotation shaft with respect to the control device. The control device may include: a device body disposed on the base; a device rotation shaft forming a rotation center of the device body; and an interlocking protrusion spaced apart from the device rotation shaft and formed integrally with the device body. The interlocking member may include: a member body rotatably provided on the seating member about an axis of the first rotation shaft and formed in a bar shape; and an interlocking guide including a hole extending in a longitudinal direction of the member body, the device rotation shaft and the interlocking protrusion being disposed in the interlocking guide.

In accordance with another aspect of the present disclosure, a seating assembly in a magnetic resonance imaging apparatus may include: a base; a seat for an object, the seat rotatably attached to the base; and a support assembly provided under the seat and configured to elastically support the seat to compensate for a weight of the object; wherein the support assembly includes: an elastic unit configured to generate an elastic force; and a control device configured to guide a movement of the elastic unit. A first end of the elastic unit may be pivotally fixed to the seat and; a second end opposite to the first end of the elastic unit may be connected to the control device. The control device may be configured to guide the second end of the elastic unit to adjust an angle between the seat and the elastic unit thereby compensating for the weight of the object.

The first end of the elastic unit may be configured to be pivotally rotatable about a first rotation shaft with respect to the seat. The second end of the elastic unit is attached to a second rotation shaft and configured to be rotatable with respect to the control device. The control device may be configured to guide the elastic unit so that the second rotation shaft moves in a circumferential direction about the first rotation shaft.

The control device may include a shaft guide configured to guide the second rotation shaft, and the shaft guide may have an arc shape in which the second rotation shaft is located at a center of a radius of curvature of the arc shape.

The control device may be interlocked with the second rotation shaft so that the shaft guide maintains the second rotation shaft, which moves, to be at the center of the radius of curvature of the shaft guide.

### BRIEF DESCRIPTION OF THE DRAWINGS

The above and/or other aspects of the disclosure will become apparent and more readily appreciated from the following description of the embodiments, taken in conjunction with the accompanying drawings of which:
FIG. 1 is a schematic view of a magnetic resonance imaging apparatus according to an embodiment of the present disclosure;
FIG. 2 is a perspective view of a seating apparatus of a magnetic resonance imaging apparatus according to an embodiment of the present disclosure;
FIG. 3 is a side view of a seating apparatus of a magnetic resonance imaging apparatus according to an embodiment of the present disclosure;
FIG. 4 is an enlarged view of a part of the configuration of a seating apparatus according to an embodiment of the present disclosure;
FIG. 5 is an exploded perspective view of a seating apparatus of a magnetic resonance imaging apparatus according to an embodiment of the present disclosure;
FIG. 6 is an exploded perspective view of a seating apparatus of a magnetic resonance imaging apparatus according to an embodiment of the present disclosure;
FIGS. 7 to 10 are views illustrating operations of a seating apparatus of a magnetic resonance imaging apparatus according to an embodiment of the present disclosure;
FIGS. 11 and 12 are views illustrating operations of a seating apparatus of a magnetic resonance imaging apparatus according to an embodiment of the present disclosure; and
FIG. 13 is a side view of a seating apparatus of a magnetic resonance imaging apparatus according to another embodiment of the present disclosure.

### DETAILED DESCRIPTION

The present specification describes the principles of the present disclosure and discloses embodiments in order to clarify the scope of the present disclosure and to enable a person skilled in the art to carry out the inventive concept of the present disclosure. The disclosed embodiments may be implemented in various forms.

Like reference numerals refer to like elements throughout this specification. This specification does not describe all components of the embodiments, and general contents in the technical field to which the present disclosure belongs or overlapping contents between the embodiments will not be described. The terms "portion," "module," "member," and "block" as used herein, may be implemented as software or hardware, and according to embodiments, a plurality of "portions," "modules," "members," or "blocks" may be implemented as a single component, or a single "portion," "module," "member," or "block" may include a plurality of components.

The image described herein may include a medical image acquired by a medical imaging apparatus, such as a magnetic resonance imaging (MRI) apparatus, a computed tomography (CT) apparatus, an ultrasonic imaging apparatus, or an x-ray imaging apparatus.

In this specification, an "object" may be a patient including a person, an animal, or a body part thereof, which is an object of photography. For example, an object may include a part of the body (organ) or a phantom.

A magnetic resonance imaging apparatus acquires a magnetic resonance (MR) signal, and reconstructs the acquired MR signal into an image. The MR signal refers to an RF signal emitted from an object.

In the magnetic resonance imaging apparatus, the main magnet generates a static magnetic field, and aligns the directions of the magnetic dipole moments of the specific atomic nuclei of the object located in the static magnetic field in the direction of the static magnetic field. The gradient magnetic field coil may apply a gradient signal to the static magnetic field to generate a gradient magnetic field, and may induce different resonance frequencies for each part of the object.

The RF coil may emit an RF signal to match the resonance frequency of a site where the image acquisition is desired. Further, as the gradient magnetic field is generated, the RF coil may receive MR signals of different resonance frequencies radiated from various sites of the object. Through these steps, the magnetic resonance imaging apparatus acquires an image from the MR signal using an image restoration technique.

Hereinafter, embodiments according to the present disclosure will be described in detail with reference to the accompanying drawings.

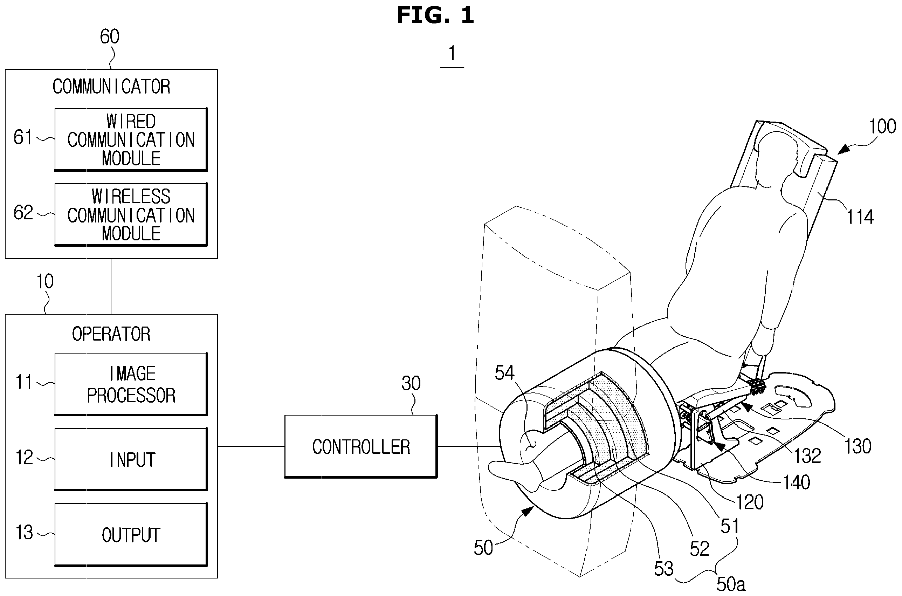

FIG. 1 is a schematic view of a magnetic resonance imaging (MRI) apparatus 1 according to an embodiment of the present disclosure.

Referring to FIG. 1, an MRI apparatus 1 may include an operator 10, a controller 30, and a scanner 50. The controller 30 may be implemented independently from the operator 10 and the scanner 50 as shown in FIG. 1. Alternatively, the controller 30 may be divided into a plurality of components and may be incorporated separately in different modules of the MRI apparatus 1. Hereinafter, each component of the MRI apparatus 1 will be described in detail.

The scanner 50 may be implemented as a body or a structure having a certain shape (for example, a body / structure having a bore shape) in which the internal space thereof is void so that an object (or a portion thereof) may be inserted into the internal space. A static magnetic field and a gradient magnetic field are formed in the internal space of the scanner 50, and an RF signal is emitted to the internal space. The internal space into which the object (or the portion thereof) is inserted may be referred to as a cavity 54.

The scanner 50 may include a magnet assembly 50a, a seating apparatus 100, and a display (not shown). The magnet assembly 50a may include a static magnetic field forming portion 51, a gradient magnetic field forming portion 52, and an RF coil portion 53. The magnet assembly 50a may be provided on the perimeter around the cavity 54. The static magnetic field forming portion 51 forms a static magnetic field for ali 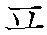 to an object according to a control signal transmitted from the controller 30 and receive an MR signal emitted from the object. The RF coil portion 53 may transmit an RF signal with a frequency equal to the frequency of precession motion to the object toward an atomic nucleus carrying out a precession motion and then stop the transmission of the RF signal, and may receive the MR signal emitted from the object.

The RF coil portion 53 may be implemented as a transmitting RF coil for generating an electromagnetic wave having a radio frequency corresponding to the type of atomic nucleus and a receiving RF coil for receiving the electromagnetic wave radiated from the atomic nucleus. Alternatively, the RF coil portion 53 may be implemented as one RF transmission/reception coil having both a transmitting function and a receiving function. In addition to the RF coil portion 53, a separate coil may be mounted on the object. For example, a head coil, a spine coil, a torso coil, a knee coil, or the like may be used as the separate coil depending on a region to be scanned on the object.

Although not shown in FIG. 1, a display may be provided on the outside and/or inside of the scanner 50. The display may be controlled by the controller 30 to provide a user or the object with information related to medical imaging or the control thereof.

The scanner 50 may also be provided with an object monitoring information obtainer (not shown) for acquiring and transmitting monitoring information related to the condition of the object. For example, the object monitoring information obtainer may obtain monitoring information on an object from a camera (not shown) that photographs a motion, a position, and the like of the object, from a respiration meter (not shown) for measuring the respiration of the object, from an ECG measuring device (not shown) for measuring the electrocardiogram of a subject, or from a body temperature measuring device (not shown) for measuring the body temperature of the object, and may transmit the monitoring information to the controller 30. Accordingly, the controller 30 may control the operation of the scanner 50 using the monitoring information on the object. Hereinafter, the controller 30 will be described. The controller 30 may control the overall operation of the scanner 50.

The controller 30 may control a sequence of signals generated inside the scanner 50. The controller 30 may also control the gradient magnetic field forming portion 52 and the RF coil portion 53 according to a pulse sequence received from the operator 10 or a designed pulse sequence.

The pulse sequence includes all information necessary for controlling the gradient magnetic field forming portion 52 and the RF coil portion 53. For example, the pulse sequence may include information on the intensity of the pulse signal applied to the gradient magnetic field forming portion 52, the duration of application, the timing of application, and the like.

The controller 30 may also control i) a waveform generator (not shown) which generates a gradient waveform, (i.e., a current pulse in accordance with the pulse sequence) and ii) a gradient amplifier (not shown) which amplifies the generated current pulse and transmits the amplified current pulse to the gradient magnetic field forming portion 52 to control the formation of the gradient magnetic field of the gradient magnetic field forming portion 52.

The controller 30 may also control the operation of the RF coil portion 53. For example, the controller 30 may supply an RF pulse of a resonance frequency to the RF coil portion 53 to emit the RF signal, and receive the MR signal received by the RF coil portion 53 from the object. At this time, the controller 30 may control the operation of a switch (for example, a transmit/receive (T/R) switch) capable of adjusting the transmission/reception direction through the control signal to control the irradiation of the RF signal and the reception of the MR signal according to an operation mode.

The controller 30 may control the movement of a seating apparatus 100 in which the object is located. Before the imaging is performed, the controller 30 may move the seating apparatus 100 in advance in accordance with the imaging region of the object. Details of the seating apparatus 100 will be described later.

The controller 30 may control the display. For example, the controller 30 may control on/off of the display through a control signal, or input from a screen or through graphical user interface displayed through the display.

The controller 30 may be implemented with i) an algorithm for controlling the operation of components in the magnetic resonance imaging apparatus 1, ii) a memory (not shown) for storing program type data, and iii) a hardware processor (not shown) for performing the above-described operation using data stored in the memory. In this case, the memory and the processor may be implemented as separate chips. Alternatively, the memory and the processor may be implemented in a single chip.

The operator 10 may control the overall operation of the magnetic resonance imaging apparatus 1. The operator 10 may include an image processor 11, an input 12, and an output 13.

The image processor 11 may store the MR signal received from the controller 30 in the memory and apply an image restoration technique using an image processor to generate image data related to the object from the stored MR signal.

For example, when k-space data is completed by filling the k-space (e.g., Fourier space or frequency space) of the memory with digital data, the operator 10 may restore the k-space data into image data by applying various image restoration techniques (for example, inverse Fourier transform of k-space data) through an image processor.

Various signal processes applied to the MR signal by the image processor 11 may be performed in parallel. For example, a plurality of MR signals received by a multichannel RF coil may be subjected to signal processing in parallel to be restored into image data. The restored image data may be stored in the memory by the image processor 11 or may be stored in an external server through a communicator 60 by the controller 30, which will be described later.

The input 12 may receive a control command related to the overall operation of the magnetic resonance imaging apparatus 1 from the user (an operator of the magnetic resonance imaging apparatus 1). For example, the input 12 may receive object information, parameter information, scan conditions, information on pulse sequences, and the like from the user. The input 12 may be implemented as a keyboard, a mouse, a trackball, a voice recognizer, a gesture recognizer, a touch screen, or the like.

The output 13 may output the image data generated by the image processor 11. The output 13 may also output a user interface (Ul) configured to allow the user to input a control command related to the magnetic resonance imaging apparatus 1. The output 13 may be implemented as a speaker, a printer, a display, or the like.

FIG. 1 illustrates a configuration in which the operator 10 and the controller 30 are embodied as separate components from each other. However, as described above, the operator 10 and the controller 30 may be included in one device. Further, the processes performed by each of the operator 10 and the controller 30 may be performed in other components. For example, the image processor 11 may convert the MR signal received by the controller 30 into a digital signal, or the controller 30 may directly convert the MR signal into a digital signal.

The magnetic resonance imaging apparatus 1 includes the communicator 60, and may be connected to an external device (not shown) (for example, a server, a medical device, a portable device (smartphone, tablet PC, wearable device, etc.)) through the communicator 60.

The communicator 60 may include one or more components which enable communication with an external device. For example, the communicator 60 may include at least one of a local communication module (not shown), a wired communication module 61, and a wireless communication module 62.

The communicator 60 may also receive a control signal and data from an external device and transmit the received control signal to the controller 30 so that the controller 30 controls the magnetic resonance imaging apparatus 1 in accordance with the received control signal.

Alternatively, the controller 30 may transmit a control signal to an external device through the communicator 60, thereby controlling the external device in accordance with the control signal of the controller 30.

For example, the external device may process data of the external device according to a control signal of the controller 30 received through the communicator 60.

The external device may be provided with a program capable of controlling the magnetic resonance imaging apparatus 1, and the program may include commands for performing part or all of the operation of the control controller 30.

The program may be installed in advance in an external device, or a user of the external device may download and install the program from a server providing the application.

Components of the magnetic resonance imaging apparatus 1 may be separately arranged in a scanning room, a machine room, and an operating room. For example, the scanner 50 may be located in the scanning room, the controller 30 may be located in the machine room, and the operator 10 may be located in the operating room. However, such arrangement may be modified as needed.

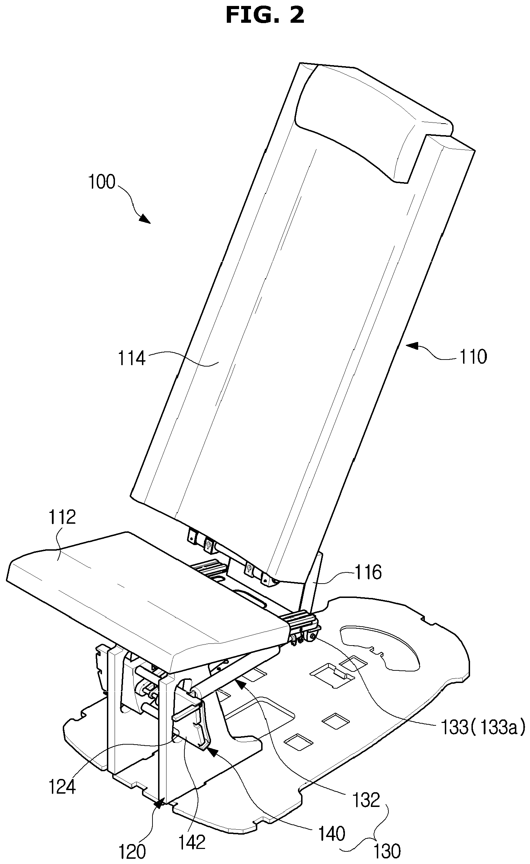

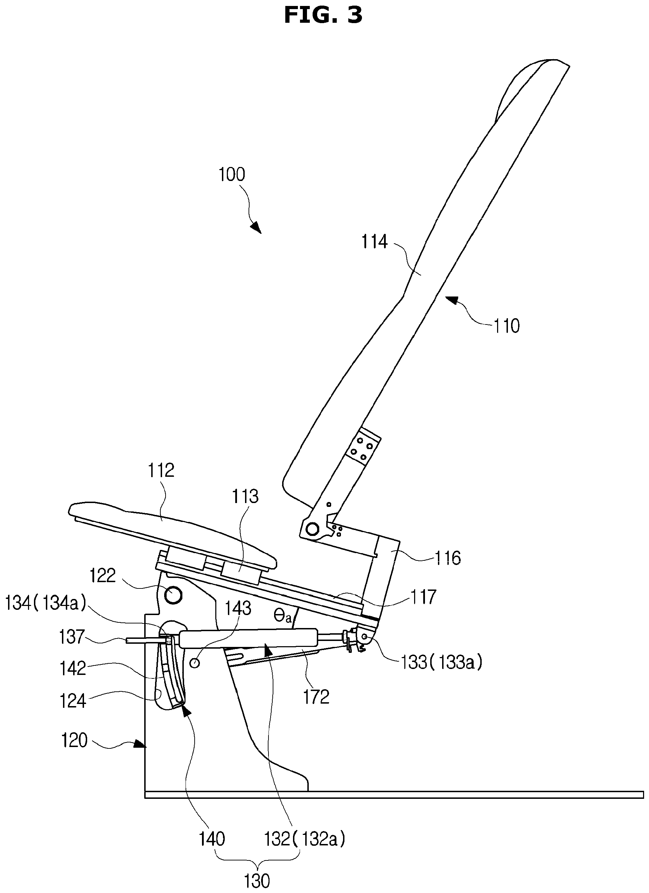

FIG. 2 is a perspective view of a seating apparatus 100 of a magnetic resonance imaging apparatus 1 according to an embodiment of the present disclosure, and FIG. 3 is a side view of a seating apparatus 100 of a magnetic resonance imaging apparatus 1 according to an embodiment of the present disclosure.

The magnetic resonance imaging apparatus 1 may include the seating apparatus 100.

The seating apparatus 100 may be configured to allow an object to be seated and adjusted in position. The seating apparatus 100 may adjust the position of the object seated on the seating apparatus 100 so that a region to be scanned of the object may be inserted into the cavity 54 or so that the region may be released to the outside of the cavity 54 by adjusting the position, angle and height of a seating member 110.

The seating apparatus 100 may include the seating member 110 on which an object is seated and a base 120 for rotatably supporting the seating member 110.

The object may be seated in the seating member 110, and the seating member 110 is configured to be rotatable with respect to the base 120 to insert the object into the cavity 54.

The seating member 110 may include a sitting plate 112 on which the object sits, and a backrest 114 on which the object sitting on the sitting plate 112 may lean. The seating member 110 is configured to allow the object to take a stable posture for the scanning. The sitting plate 112 and the backrest 114 may be configured to vary the angle between each other.

The seating member 110 may include a connecting member 116 for connecting the sitting plate 112 and the backrest 114. The connecting member 116 may be configured to support the sitting plate 112 and the backrest 114. The connecting member 116 supports the sitting plate 112 and the backrest 114 so that the angle between the sitting plate 112 and the backrest 114 may be varied or adjusted.

The sitting plate 112 of the seating member 110 may be provided to be translated in the front-rear direction. A sliding rail 117 extending in the front-rear direction may be provided at an upper portion of the connecting member 116 to guide the sitting plate 112. The sitting plate 112 may include a sitting plate moving portion 113 which is engaged with the sliding rail 117 at a lower surface thereof so as to move along the sliding rail 117.

The base 120 is configured to support the seating member 110.

The base 120 may also be connected with the magnet assembly 50a including the static magnetic field forming portion 51, the gradient magnetic field forming portion 52, and the RF coil portion 53. The base 120 may be disposed adjacent to the magnet assembly 50a.

The base 120 may be provided on the ground/floor to support the seating member 110. The base 120 may rotatably support the seating member 110 via the connecting member 116. Specifically, the base 120 may rotatably support the connecting member 116 of the seating member 110 so that the seating member 110 may be tilted backward (in a clock-wise direction). The base 120 may include a rotation shaft 122 and the seating member 110 may be configured to rotate about the rotation shaft 122.

The rotation shaft 122 is provided on a front side of the seating member 110, and the seating member 110 is configured to be tilted rearward (in a clock-wise direction) through the above-described configuration.

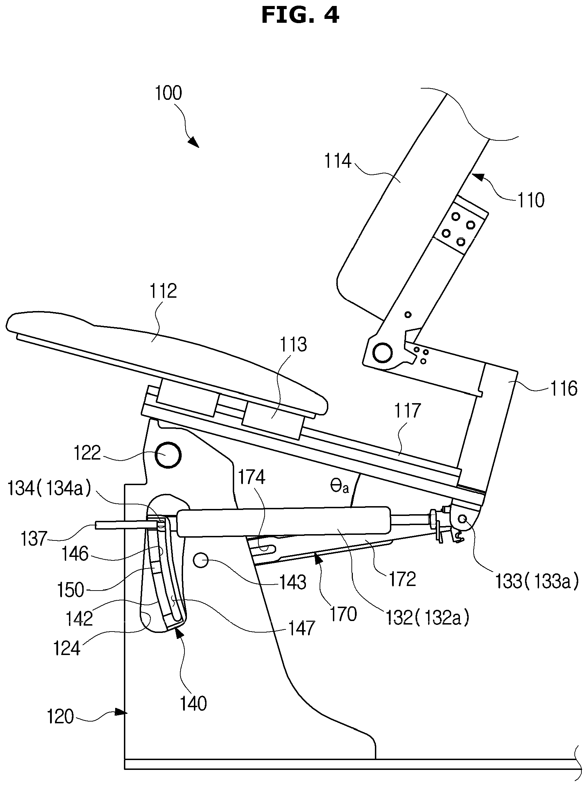

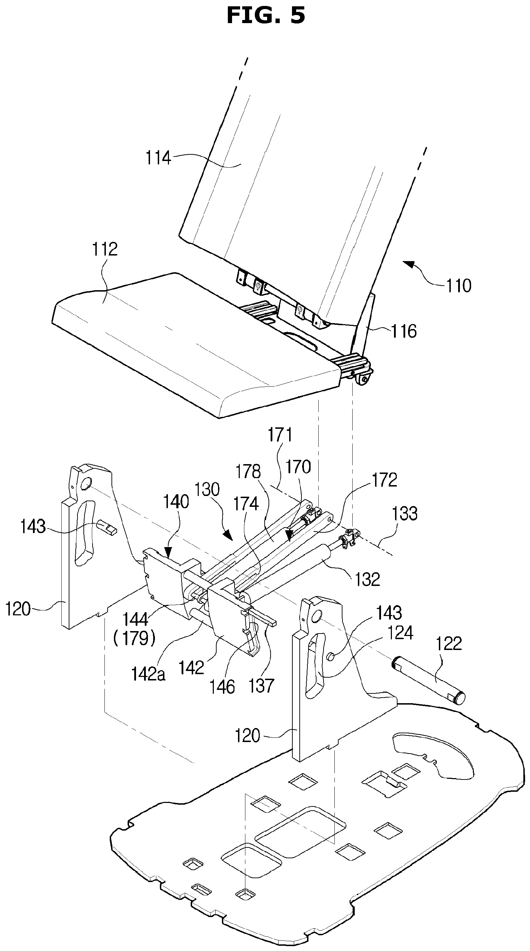

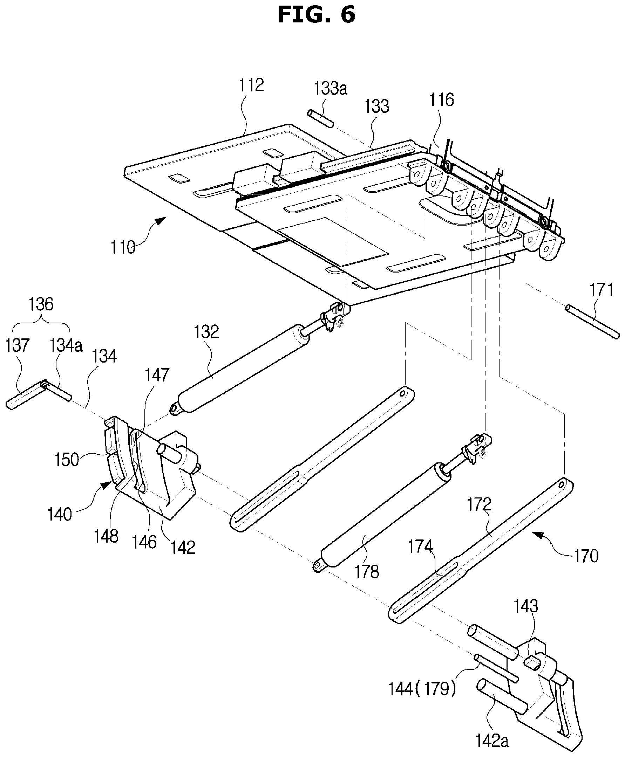

FIG. 4 is an enlarged view of a portion of seating apparatus 100 according to an embodiment of the present disclosure, FIG. 5 is an exploded perspective view of a seating apparatus 100 of a magnetic resonance imaging apparatus 1 according to an embodiment of the present disclosure, and FIG. 6 is an exploded perspective view of a portion of a seating apparatus 100 of a magnetic resonance imaging apparatus 1 according to an embodiment of the present disclosure.

The seating apparatus 100 may include an elastic assembly 130 (FIG. 5). The elastic assembly 130 may be configured to support the seating member 110 on the base 120. The elastic assembly 130 may elastically support the seating member 110 on the base 120 to compensate for a load of the object in the gravity direction.

The elastic assembly 130 may include an elastic unit 132 and a control device 140.

The elastic unit 132 is configured to elastically support the seating member 110. One end of the elastic unit 132 is connected to the seating member 110 and is configured to generate an elastic force. The base 120 may be configured to rotatably support a front portion of the seating member 110 and the elastic unit 132 may be configured to elastically support a rear portion of the seating member 110 (where the backrest 114 is disposed).

The elastic unit 132 may be configured to support the seating member 110 on the base 120. The elastic unit 132 may adjust the angle of the seating member 110 by compensating for the load generated based on the weight of the object. The type of the elastic unit 132 is not limited, and in the present embodiment, the elastic unit 132 may include a gas spring.

The control device 140 is provided at one side of the elastic unit 132 to adjust the angle between the seating member 110 and the elastic unit 132. The control device 140 may be provided at the base 120 and is configured to change the position of one end of the elastic unit 132 so that the elastic unit 132 may adjust the gravity direction elastic force with respect to the seating member 110. The control device 140 may be included in the configuration of the base 120.

The control device 140 may be configured to adjust the angle between the seating member 110 and the elastic unit 132. The control device 140 is configured to adjust the placement angle of the elastic unit 132 according to the weight of the object to be seated on the seating member 110. That is, the control device 140 is configured to guide the rotational movement of the elastic unit 132 and fix the position of the adjusted elastic unit 132, in order to change the angle of the elastic unit 132 based on the object to be seated on the seating member 110.

The elastic unit 132 is configured such that opposite ends of the elastic unit 132 are rotatable with respect to the seating member 110 and the base 120, respectively, so that the seating member 110 and the base 120 may have different arranging angles from each other. The elastic unit 132 may include a first rotation shaft 133 provided at one end of the elastic unit 132 to be rotatable with respect to the seating member 110, and a second rotation shaft 134 provided at the other end of the elastic unit 132 to be rotatable with respect to the control device 140. The first rotation shaft 133 is positioned at a position fixed to the seating member 110 and the second rotation shaft 134 is provided such that the position in the base 120 may be rotatably adjusted by the control device 140. The elastic unit 132 may include a fixed shaft 133a that forms the first rotation shaft 133. The elastic unit 132 may include a moving shaft 134a that forms the second rotation shaft 134. The fixed shaft 133a and the moving shaft 134a may be aligned such that the axial directions of the first and second rotation shafts 133 and 134 are in a longitudinal direction of the seating member 100.

The control device 140 is configured to guide the elastic unit 132 to move the second rotation shaft 134 in a circumferential direction about the first rotation shaft 133. That is, the elastic unit 132 is configured to be rotatable in the circumferential direction about the first rotation shaft 133.

The control device 140 may include a device body 142 and a shaft guide 146 formed in the device body 142.

The device body 142 constitutes a body of the control device 140 and may be formed on the base 120. A pair of the device bodies 142 may be provided, and the pair of device bodies 142 may be connected to each other by a device connecting shaft 142a. A pair of the elastic units 132 may be connected to the pair of device bodies 142, respectively. Although only one of the elastic units 132 is illustrated and described in the present embodiment, the present disclosure is not limited thereto. For example, a pair of the elastic units 132 may be provided and connected to each of the device bodies 142.

The shaft guide 146 is provided in the device body 142 and may be provided to guide the rotational movement of the elastic unit 132 being pivoted about the first rotation shaft 133. The shaft guide 146 may be provided to form an arc about the first rotation shaft 133. The shaft guide 146 may form an arc-shaped adjustment path 147 through which the moving shaft 134a moves. The moving shaft 134a moves along the arc-shaped adjustment path 147 and may change the arrangement angle of the elastic unit 132 according to the weight of the object.

The shaft guide 146 may include a stopper surface 148. The stopper surface 148 is provided to constitute an outer surface of the arc-shaped adjustment path 147. The stopper surface 148 is provided to restrict movement of the moving shaft 134a in the radial direction with respect to the first rotation shaft 133. That is, the stopper surface 148 is provided to restrict the rotation by more than an threshold angle set by the seating member 110.

The control device 140 is configured to be able to fix the position of the moving shaft 134a of the elastic unit 132 so that the elastic unit 132 may be moved along the shaft guide 146 and fixed at a certain point along the adjustment path 147.

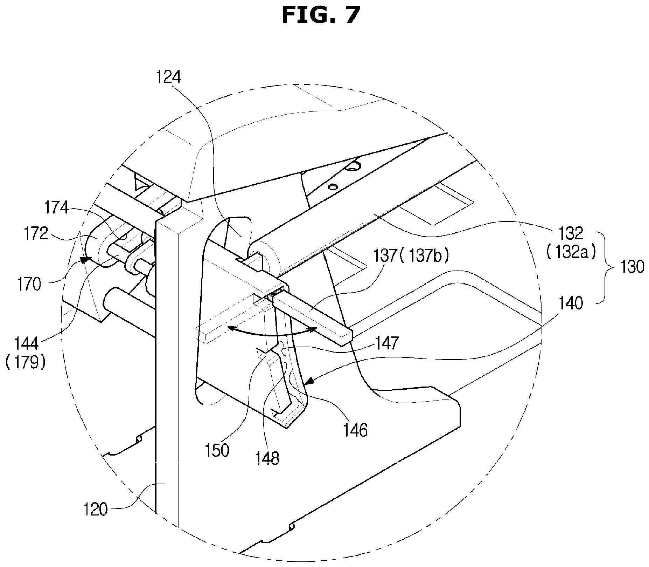

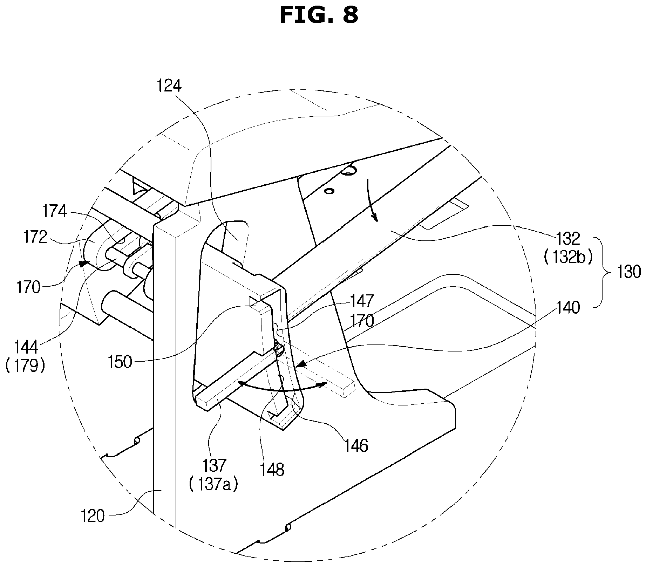

The elastic unit 132 may include a unit moving member 136. The unit moving member 136 may include the moving shaft 134a described above and a control handle 137 connected to the moving shaft 134a. The control handle 137 is rotatably coupled to the moving shaft 134a and is provided to move between a restriction position 137a (refer to FIG. 8) and a movement position 137b (refer to FIG. 7). That is, the control handle 137 may move between the restriction position 137a where the elastic unit 132 is restricted to the control device 140 so that the rotational movement of the elastic unit 132 is restricted and the movement position 137b where the restricting of the elastic unit 132 is released so that the moving shaft 134a of the elastic unit 132 may move along the shaft guide 146.

The control device 140 may include a handle fixing portion 150 for fixing the control handle 137. The handle fixing portion 150 may be provided to extend from the shaft guide 146. Specifically, the handle fixing portion 150 is provided to extend from the shaft guide 146 in a radial direction so that the control handle 137 may be fixed to the handle fixing portion 150. The handle fixing portion 150 may be provided to form a concave groove on one side of the device body 142 so that the control handle 137 may be inserted in the handle fixing portion 150. The handle fixing portion 150 is provided to restrict the moving shaft 134a from moving along the shaft guide 146 by fixing the control handle 137. When the control handle 137 is in the movement position 137b, the moving shaft 134a is movable along the shaft guide 146. When the control handle 137 is in the restriction position 137a, the moving shaft 134a is positioned at one point of the shaft guide 146 and the control handle 137 is rotated with respect to the moving shaft 134a to be inserted into the handle fixing portion 150, so that the movement of the elastic unit 132 may be restricted.

A plurality of the handle fixing portions 150 may be provided. The plurality of handle fixing portions 150 may be disposed to be spaced apart from each other along a longitudinal direction of the shaft guide 146. The plurality of handle fixing portions 150 may be formed in a radial direction from the shaft guide 146. The arrangement angle of the elastic units 132 may be changed according to the weight of the object by positioning the unit moving member 136 of the elastic unit 132 in any one of the plurality of handle fixing portions 150.

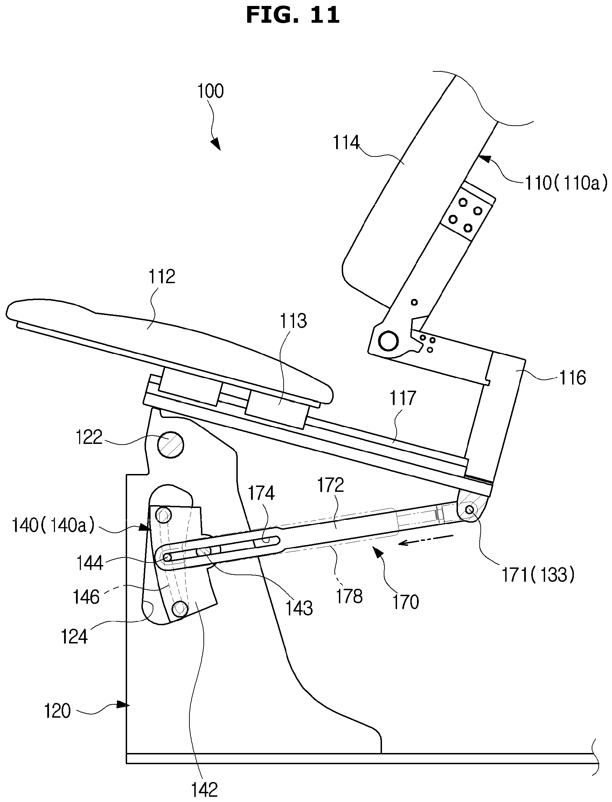

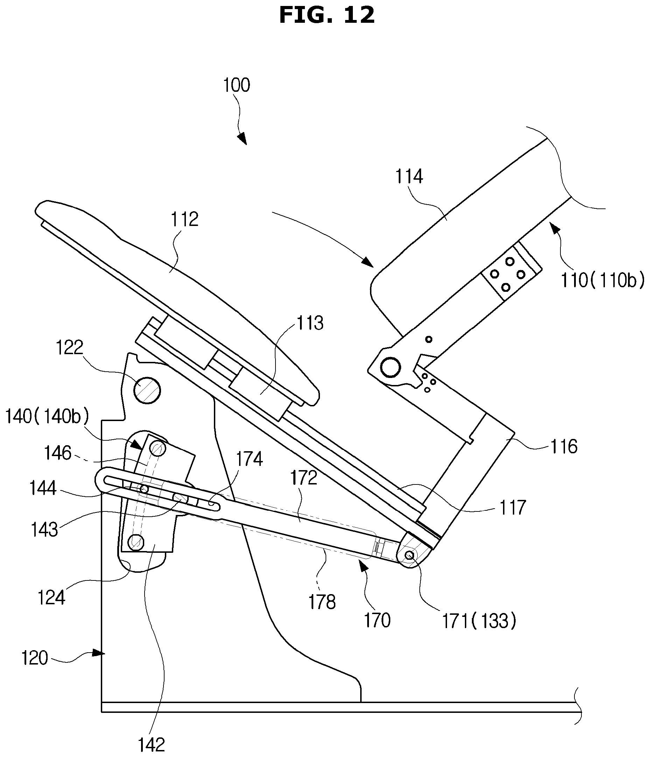

The control device 140 may be rotatably provided on the base 120. The seating member 110 may move between a first position 110a (refer to FIG. 11) and a second position 110b (refer to FIG. 12) that is tilted backward from the first position 110a. For convenience of explanation, it has been described that the seating member 110 moves between the two positions, but the seating member 110 does not move only between the two positions, and may move between various angles. The control device 140 may move between a first device position 140a (refer to FIG. 11) corresponding to the first position 110a of the seating member 110 and a second device position 140b (refer to FIG. 12) rotated from the first device position 140a. Although it has been described that the control device 140 moves between the first and second device positions 140a and 140b for convenience of explanation, as in the case of the seating member 110, the control device 140 does not move only between the two positions, and may move between various angles.

The control device 140 may move between the first and second device positions 140a and 140b in conjunction with the movement of the seating member 110 when the seating member 110 moves between the first and second positions 110a and 110b.

The control device 140 may be configured to be rotatable relative to the base 120 about a device rotation shaft 143 formed in the device body 142. The base 120 may include a device movement portion 124 that constitutes a rotational space of the control device 140. The device movement portion 124 may also serve as a stopper of rotational movement of the control device 140. Specifically, the device movement portion 124 serves as a stopper in the movement of the device connecting shaft 142a of the control device 140, thereby restricting the movement of the control device 140.

The seating apparatus 100 may include an interlocking member 170 so that the control device 140 may rotate in conjunction with the rotation of the seating member 110.

One end of the interlocking member 170 is connected to the seating member 110, and the other end of the interlocking member 170 is connected to the control device 140. The interlocking member 170 is rotatably connected to the seating member 110 about an interlocking shaft 171 formed at one end thereof. That is, the interlocking member 170 is pivoted with respect to the interlocking shaft 171. The interlocking member 170 is configured to rotate together with the rotation of the seating member 110 by the rotation of the seating member 110 being transmitted to the control device 140. The interlocking shaft 171 may be positioned along the same rotational axis of the first rotation shaft 133 together with the fixed shaft 133a.

The interlocking member 170 may include a member body 172 and an interlocking guide 174 formed in the member body 172. The member body 172 may have a bar shape and the interlocking guide 174 may be provided to form a bar-shaped hole or an elongated slot in the member body 172. The member body 172 may be rotatably formed with the seating member 110 about the same rotational axis as the first rotation shaft 133. The interlocking guide 174 may be formed in the same longitudinal direction as the member body 172.

The device rotation shaft 143 of the control device 140 and an interlocking protrusion 144 formed on the device body 142 are positioned together in the interlocking guide 174. The device rotation shaft 143 and the interlocking protrusion 144 may be arranged so as to be located on the same line as a longitudinal direction of the interlocking member 170. The rotation of the device rotation shaft 143 and the movement of the interlocking protrusion 144 is restricted by the interlocking guide 174.

The device rotation shaft 143 forms a rotation center of the interlocking member 170. Because the device rotation shaft 143 is not located at a position fixed to the interlocking member 170, but is located in a straight line section of the bar-shaped interlocking guide 174, the rotation center of the interlocking member 170 is moved in accordance with the change of the position of the device rotation shaft 143 in the interlocking guide 174. Because the movement of the interlocking protrusion 144 together with the device rotation shaft 143 is restrained by the interlocking guide 174, the interlocking protrusion 144 rotates about the device rotation shaft 143 by the rotation of the interlocking member 170. Because the interlocking protrusion 144 is integrally formed with the control device 140, the control device 140 rotates together with the movement of the interlocking protrusion 144.

With the above configuration, when the seating member 110 moves from the first position 110a to the second position 110b, the control device 140 is rotated together with the interlocking member 170 from the first device position 140a to the second device position 140b by the rotation operation of the interlocking member 170. Accordingly, the shaft guide 146 may maintain a center of the radius of curvature to be the first rotation shaft 133. That is, as shown in the embodiments of FIGS. 11 and 12, the first rotation shaft 133 is maintained at the center of the shaft guide 146 after the seating member 110 moves from the first position 110a to the second position 110b.

The seating apparatus 100 may include a hydraulic stopper 178.

The hydraulic stopper 178 may be configured to support the seating member 110 on the base 120. The hydraulic stopper 178 may prevent the seating apparatus 100 from being damaged due to a suddenly changing load by acting as a damper when the object is seated on the seating member 110. In detail, one end of the hydraulic stopper 178 may be connected to the connecting member 116 of the seating member 110 and the other end of the hydraulic stopper 178 may be connected to the base 120. Specifically, the other end of the hydraulic stopper 178 may be connected to a stopper connecting portion 179 of the control device 140 located on the base 120. In this embodiment, the stopper connecting portion 179 and the interlocking protrusion 144 are configured as the same shaft, but the present disclosure is not limited thereto, and the stopper connecting portion 179 and the interlocking protrusion 144 may be arranged separately.

Hereinafter, the operation of a magnetic resonance imaging apparatus 1 according to an embodiment of the present invention will be described.

FIGS. 7 to 10 are views illustrating operations of a seating apparatus 100 of a magnetic resonance imaging apparatus 1 according to an embodiment of the present disclosure.

First, the operation of the elastic assembly 130 capable of varying the elastic force in the direction of gravity according to the weight of an object will be described.

For convenience of explanation, the object includes first and second objects. The second object may be heavier than the first object.

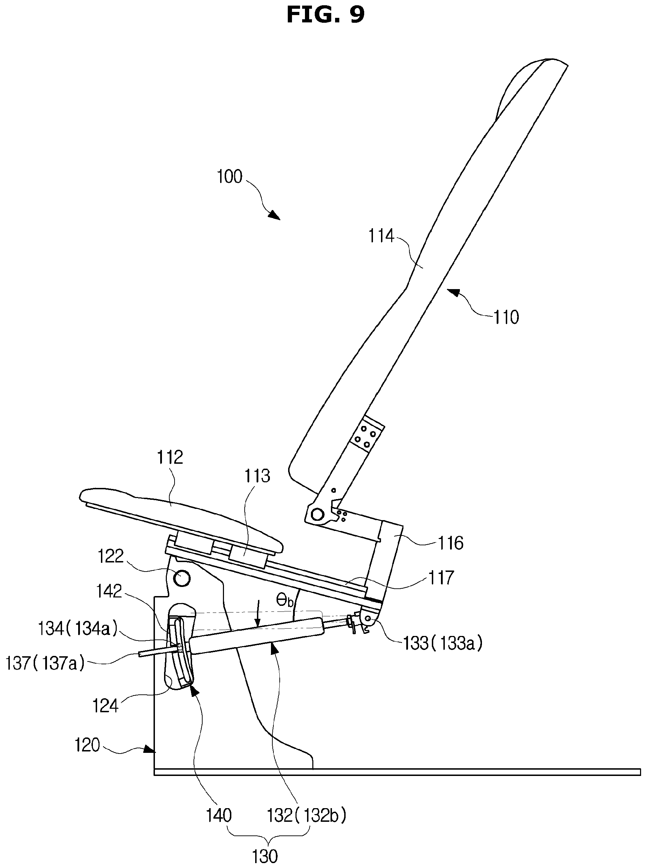

The description will be made with reference to FIGS. 3 and 4. Although the first and second objects are not shown, FIGS. 3 and 4 illustrate a seating apparatus 100 on which the first object is placed, and FIGS. 8 and 9 illustrate a seating apparatus 100 on which the second object is seated.

When the first object is seated on the seating member 110, the elastic unit 132 may be located at a first unit position 132a (refer to FIGS. 3 and 4) that is spaced apart from the seating member 110 by a first angle θa (refer to FIGS. 3 and 4). The elastic force of the elastic unit 132 located at the first unit position 132a in the gravitational direction may be offset by the load of the first object.

With the above configuration, even when the first object is placed on the seating member 110, the operator who operates the magnetic resonance imaging apparatus 1 may rotate the seating member 110 with respect to the base 120 without using a large force.

When the second object is seated on the seating member 110, the elastic unit 132 may be located at a second unit position 132b (refer to FIG. 9) that is spaced apart from the seating member 110 by a second angle θb (refer to FIG. 9). The elastic force of the elastic unit 132 located at the second unit position 132b in the gravitational direction may be offset by the load of the second object. The second angle θb is larger than the first angle θa, and the second rotation shaft 134 at the second unit position 132b is positioned lower than the second rotation shaft 134 at the first unit position 132a.

With the above configuration, even when the second object is placed on the seating member 110, the operator who operates the magnetic resonance imaging apparatus 1 may be able to rotate the seating member 110 with respect to the base 120 without using a large force.

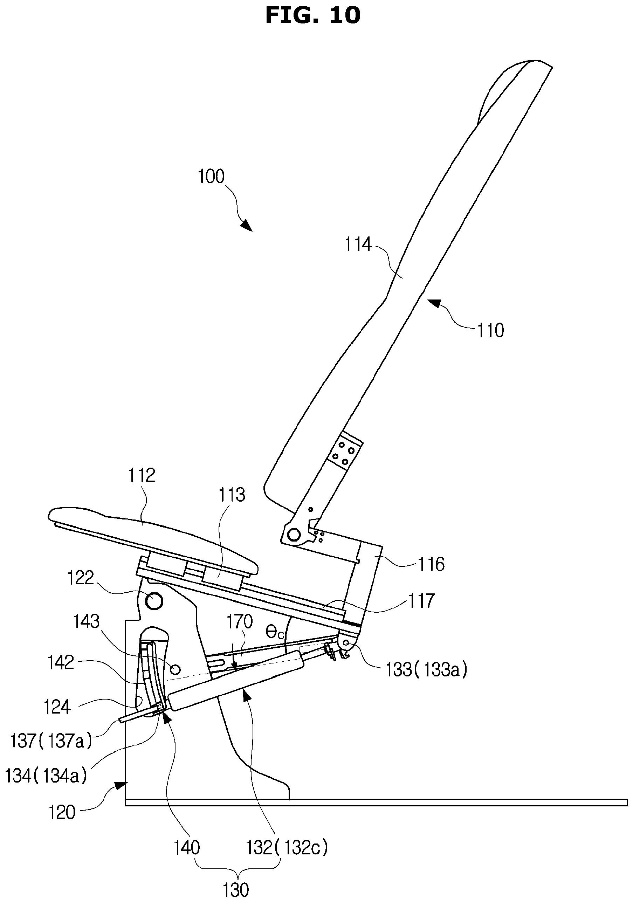

FIG. 10 illustrates a case where a third object, which is heavier than the second object, is seated on the seating apparatus 100.

When the third object is seated on the seating member 110, the elastic unit 132 may be located at a third unit position 132c (refer to FIG. 10) that is spaced apart from the seating member 110 by a third angle θc (refer to FIG. 10). The elastic force of the elastic unit 132 located at the third unit position 132c in the gravitational direction may be offset by the load of the third object. The third angle θc is larger than the first and second angles θa and θb, and the second rotation shaft 134 at the third unit position 132c is positioned lower than the second rotation shaft 134 at the first unit position 132a and the second rotation shaft 134 at the second unit position 132b.

The operation of moving the elastic unit 132 amongst the first unit position 132a, the second unit position 132b and the third unit position 132c will be described below. As shown in FIG. 7, first, the control handle 137 is deviated from the handle fixing portion 150 in the control device 140 (i.e., from the restriction position 137a to the movement position 137b). Thereby, the moving shaft 134a of the elastic unit 132 becomes movable/rotatable along the shaft guide 146. Next, as shown in FIG. 8, after the moving shaft 134a is moved so that the angle between the elastic unit 132 and the seating member 110 becomes the second angle θb from the first angle θa, the control handle 137 is inserted into the handle fixing portion 150 (i.e., from the movement position 137b to the restriction position 137a). When the control handle 137 is inserted into the handle fixing portion 150, the moving shaft 134a is restrained from moving and the elastic unit 132 is fixed to the second unit position 132b. The operation in which the moving shaft 134a moves from the second unit position 132b to the third unit position 132c is the same as the above-described operation.

With the above operation, even when the weight of the objects is different, the load compensation may be made differently through the operation of the elastic assembly 130, so that the operation of the seating apparatus 100 may be facilitated.

Although the operation of adjusting the elastic assembly 130 before the first, second, and third objects are seated on the seating apparatus 100 has been described in the present embodiment, the elastic assembly 130 may also be adjusted after the objects are seated on the seating apparatus 100.

For convenience of description, the elastic unit 132 is limited to be placed in the first, second, and third unit positions 132a, 132b, and 132c, but is not limited thereto. For example, the elastic unit 132 may be provided to be placed at a position corresponding to any one of the handle fixing portions 150 provided at two or more positions.

The interlocking operation of the control device 140 according to the rotation of the seating member 110 will be described below.

When the seating member 110 moves to the second position 110b (see FIG. 12) which is tilted at a predetermined angle from the first position 110a (see FIG. 11), the interlocking shaft 171 disposed integrally with the seating member 110 moves together with the seating member 110.

As described earlier, the device rotation shaft 143 and the interlocking protrusion 144 are provided to move along the interlocking guide 174 of the interlocking member 170. With this configuration, the interlocking member 170 is rotated about the device rotation shaft 143 by the movement of the interlocking shaft 171, and the interlocking protrusion 144 is also moved in the interlocking guide 174 of the interlocking member 170.

Through this operation, the control device 140 rotates in conjunction with the rotation of the seating member 110 and may maintain a center of curvature radius of the shaft guide 146 of the control device 140 to be the first rotation shaft 133.

Hereinafter, a magnetic resonance imaging apparatus according to another embodiment of the present disclosure will be described. In the present embodiment, the same components as those described above will not be described.

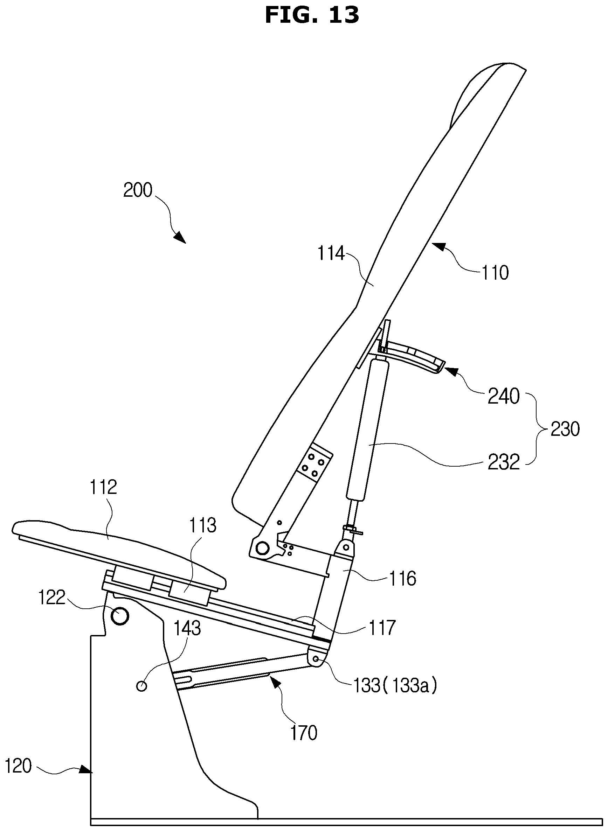

FIG. 13 is a side view of a seating apparatus 200 of a magnetic resonance imaging apparatus 1 according to another embodiment of the present disclosure.

A seating apparatus 200 may include the seating member 110, the base 120, and an elastic assembly 230.

The elastic assembly 230 may include an elastic unit 232 and a control device 240.

The elastic unit 232 is configured to elastically support the backrest 114 of the seating member 110. The elastic unit 232 may be configured to be disposed between the connecting member 116 of the seating member 110 and the backrest 114 to support the backrest 114. The elastic unit 232 may easily adjust the angle between the sitting plate 112 and the backrest 114 of the seating member 110 by compensating for the load according to the weight of the object. The type of the elastic unit 232 is not limited, and in the present embodiment, the elastic unit 132 may include a gas spring.

The control device 240 is provided at one end side of the elastic unit 232 to adjust the angle of the elastic unit 232. The control device 240 may be provided on the back surface of the backrest 114 of the seating member 110 and is configured to change the position of one end of the elastic unit 232 so that the elastic unit 232 may change the gravity direction elastic force with respect to the backrest 114. The control device 240 may be included in the configuration of the seating member 110.

The control device 240 may be configured to adjust the angle between the sitting plate 112 and the backrest 114 of the seating member 110. The control device 240 is configured to adjust the placement angle of the elastic unit 232 according to the weight of the object to be seated on the seating member 110. That is, the control device 240 is configured to guide the rotational movement of the elastic unit 232 and fix the position of the moved elastic unit 232, in order to change the angle of the elastic unit 232. The operation of moving the elastic unit 232 in the control device 240 may be the same as the operation of the previous embodiment.

As is apparent from the above, according to an aspect of the present disclosure, a posture of a patient can be stably maintained.

According to another aspect of the present disclosure, a posture of a patient or a measurement angle can be easily changed.

According to another aspect of the present disclosure, an angle of the seating apparatus on which a patient is seated can be changed with a small force.

Although embodiments of the present disclosure have been shown and described, it would be appreciated by those skilled in the art that changes may be made in the inventive concept disclosed in these embodiments without departing from the principles and spirit of the disclosure in the scope of which is defined in the claims and their equivalents.

## Claims

1. A magnetic resonance imaging apparatus comprising:
a magnet assembly;
a base disposed adjacent to the magnet assembly;
a seating member on which an object is to be seated and configured to be rotatably disposed on the base; and
an elastic assembly configured to elastically support the seating member on the base to compensate for a load of the object;
wherein the elastic assembly comprises:
an elastic unit having a first end connected to the seating member and configured to generate an elastic force; and
a control device disposed on the base and configured to guide a second end opposite to the first end of the elastic unit to adjust an angle between the seating member and the elastic unit.

2. The magnetic resonance imaging apparatus according to claim 1, wherein the control device is configured to adjust the angle between the seating member and the elastic unit according to a weight of the object.

3. The magnetic resonance imaging apparatus according to claim 1, wherein:
the elastic unit is configured to be rotatable about a first rotation shaft with respect to the seating member and to be rotatable about a second rotation shaft with respect to the control device, and
the control device is configured to guide the elastic unit so that the second rotation shaft moves in a circumferential direction about the first rotation shaft.

4. The magnetic resonance imaging apparatus according to claim 3, wherein:
the elastic unit includes a moving shaft forming the second rotation shaft, and
the control device comprises:
a device body disposed on the base; and
a shaft guide formed in the device body to guide the moving shaft.

5. The magnetic resonance imaging apparatus according to claim 4, wherein:
the elastic unit further comprises a control handle connected to the moving shaft, and
the control device further comprises a plurality of handle fixing portions extending from the shaft guide and into which the control handle is fixed to restrict a movement of the moving shaft.

6. The magnetic resonance imaging apparatus according to claim 5, wherein the plurality of handle fixing portions are formed as slots in the device body and disposed to be spaced apart from one another.

7. The magnetic resonance imaging apparatus according to claim 4, wherein the shaft guide is formed in a shape of an arc in which the first rotation shaft is a center of the radius of curvature.

8. The magnetic resonance imaging apparatus according to claim 4, wherein:
the first rotation shaft moves together with the seating member, and
the control device is configured to rotate in conjunction with a movement of the first rotation shaft.

9. The magnetic resonance imaging apparatus according to claim 8, wherein the control device is interlocked with the first rotation shaft so that the shaft guide maintains the first rotation shaft, which moves, to be at a center of a radius of curvature of the shaft guide.

10. The magnetic resonance imaging apparatus according to claim 8, wherein:
the control device comprises:
a device rotation shaft attached to the base and forming a rotation center of the device body; and
an interlocking protrusion spaced apart from the device rotation shaft and formed integrally with the device body,
the magnetic resonance imaging apparatus further comprises an interlocking member having one end connected to the seating member so as to be rotatable about an axis of the first rotation shaft, a rotation of the interlocking member being restricted by the device rotation shaft and the interlocking protrusion, and
the interlocking member is configured to transmit a force generated by a movement of the seating member to the interlocking protrusion to rotate the control device.

11. The magnetic resonance imaging apparatus according to claim 10, wherein the interlocking member comprises:
a member body rotatably provided on the seating member about the axis of the first rotation shaft and formed in a bar shape; and
an interlocking guide comprising a hole extending in a longitudinal direction of the member body, the device rotation shaft and the interlocking protrusion being disposed in the interlocking guide.

12. The magnetic resonance imaging apparatus according to claim 4, wherein the shaft guide comprises a stopper surface for restricting a movement of the moving shaft in a radial direction with respect to the first rotation shaft.

13. The magnetic resonance imaging apparatus according to claim 1, wherein:
the seating member comprises:
a sitting plate on which the object sits;
a backrest disposed to form a predetermined angle with the sitting plate; and
a connecting member connecting the sitting plate and the backrest, and
the first rotation shaft is provided in the connecting member.

14. The magnetic resonance imaging apparatus according to claim 1, wherein:
the base is configured to rotatably support a first portion of the seating member, and
the elastic unit is configured to elastically support a second portion opposite to the first portion of the seating member.

15. The magnetic resonance imaging apparatus according to claim 1, wherein the elastic unit comprises a gas spring.
